(19) Europäisches Patentamt
European Patent Office
Office européen des brevets

(11) Numéro de publication: 0 000 679
B1

(12) **FASCICULE DE BREVET EUROPEEN**

(45) Date de publication du fascicule de brevet: 24.06.81

(51) Int. Cl.³: C 07 D 215/44 //A61K31/47

(21) Numéro de dépôt: 78400060.6

(22) Date de dépôt: 18.07.78

(54) **Nouveau procédé de préparation de la glafénine.**

(30) Priorité: 26.07.77 FR 7722900

(43) Date de publication de la demande:
07.02.79 Bulletin 79/3

(45) Mention de la délivrance du brevet:
24.06.81 Bulletin 81/25

(84) Etats Contractants Désignés:
BE CH DE GB LU NL SE

(56) Documents cités:
CHIMIE THERAPEUTIQUE, 1, 65—70 (1966).

(73) Titulaire: PIERRE FABRE S.A.
125, rue de la Faisanderie
F-75116 Paris (FR)

(72) Inventeur: Casadio, Sylvano
via Tantardini 15
20136 Milan (IT)
Inventeur: Cousse, Henri, Dr.
Foun de las Nibios Chemin de Lastinos
F-81100 Castres (FR)
Inventeur: Hascoet, Pierre
Chemin de Nottes
F-81600 Gaillac (FR)
Inventeur: Mouzin, Gilbert, Dr.
21, rue Sainte Foy
F-81100 Castres (FR)

(74) Mandataire: Corre, Jacques Denis Paul et al,
Cabinet Regimbeau 26, Avenue Kléber
F-75116 Paris (FR)

EP 0 000 679 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveau procédé de préparation de la glafénine

La présente invention, réalisée au Centre de Recherche Pierre FABRE concerne un nouveau procédé de préparation de l'$\alpha$-monoglycéride de la 4(2' - carboxyphénylamino) - 7 - chloro - quinoléine de formule:

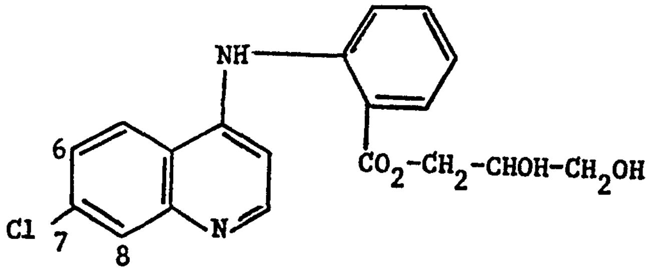

(I)

Ce composé chimique est connu pour ses propriétés antalgiques; il est utilisé comme principe actif dans de nombreuses spécialités pharmaceutiques. Cette molécule sera mentionnée, ci-après, par sa dénomination commune internationale : glafénine.

Les procédés de synthèse de la technique antérieure sont au nombre de trois:

1) *La méthode de A. ALLAIS et G. ROUSSEAU* (ROUSSEL UCLAF) décrite dans le brevet belge No. 636.381.

La glafénine (I) est préparée par condensation en milieu chlorhydrique de la dichloro - 4,7 - quinoléine (II) avec l'anthranilate de méthyle (III), cet ester méthylique intermédiaire est ensuite transestérifié par l'acétonide de glycéryde (V) et enfin l'hydrolyse acide du composé (VI) conduit à la glafénine (I) selon le schéma réactionnel:

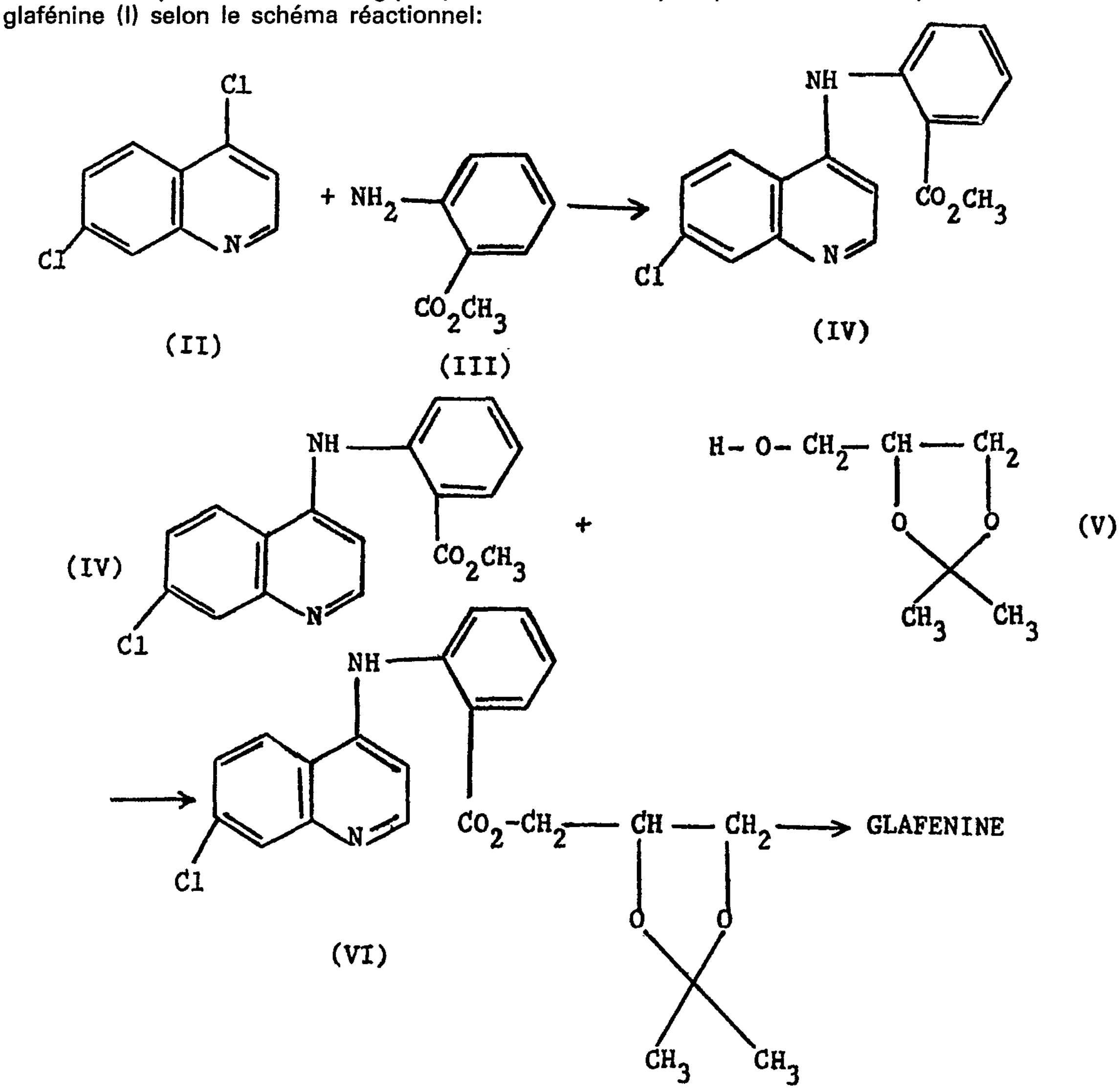


(II) (III) (IV)

(IV) (V)

(VI)

2) *Le procédé décrit dans le brevet français numéro 1.421.229.*

Procédé dans lequel on estérifie le chlorure de l'acide o-nitrobenzoïque par l'acétonide de glycéryle; le produit obtenu étant, après réduction du groupe nitro, condensé avec la 4,7 - dichloro - quinoléine; on obtient ainsi un composé qui, par hydrolyse acide, fournit la glafénine selon le schéma:

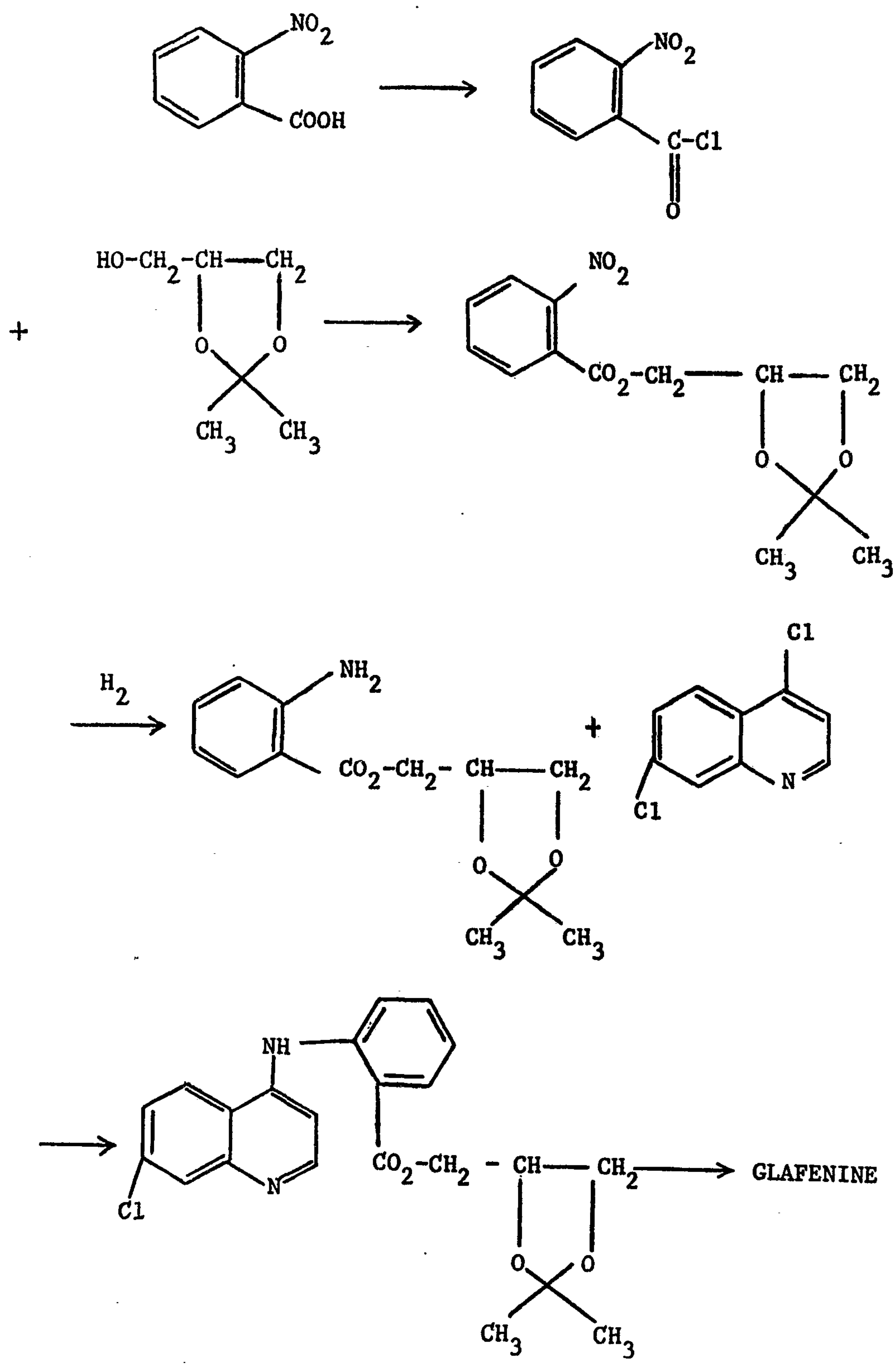

3) *La méthode de MILDNER PAVAO et MIHALIC MLADEN* décrite dans le brevet allemand No. 2.251.646.

On condense l'ortho - chloro - benzoate de glycéryle sur la 4 - amino - 7 - chloro - quinoléine:

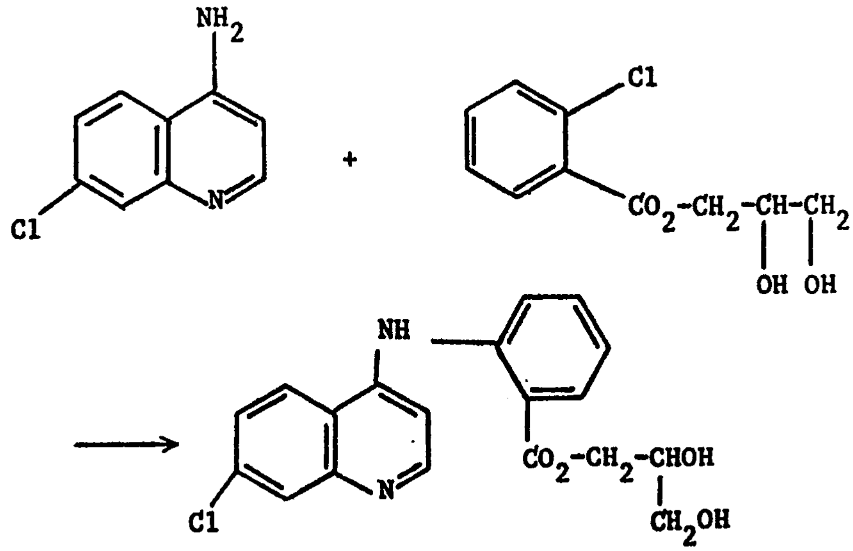

Ces procédés présentent tous de nombreux inconvénients car, soit ils utilisent des produits de départ coûteux car délicats à synthétiser, soit ils multiplient les étapes réactionnelles et, par là, augmentent le coût du procédé tout en diminuant le rendement.

La présente invention concerne un procédé très simple qui permet de préparer la glafénine dans des conditions très économiques. Pour cela, on prépare la glafénine par condensation de l'anthranilate de glycéryle de formule:

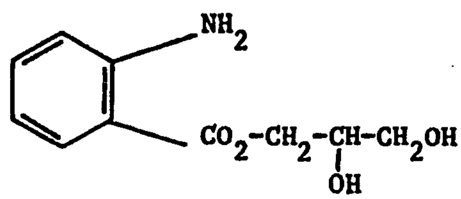

sur la 4,7 - dichloro - quinoléine:

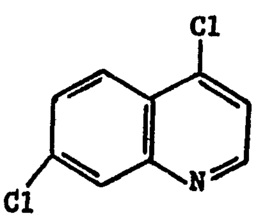

Cette condensation est conduite de préférence en milieu chlorhydrique.

On opère de préférence à une température comprise entre 60 et 90°C et en général à 80°C pendant un temps de l'ordre de 30 minutes à une heure.

La glafénine obtenue est séparée du milieu réactionnel par des procédés connus, on peut en particulier la précipiter par neutralisation du milieu réactionnel.

La glafénine brute obtenue peut être purifiée par exemple par recristallisation dans un solvant ou mélange de solvant organique tel que le mélange chloroforme-éthanol.

L'anthranilate de glycéryle est lui-même préparé selon l'invention par condensation du glycérol sur l'anhydride isatoïque en milieu basique comme agent de condensation.

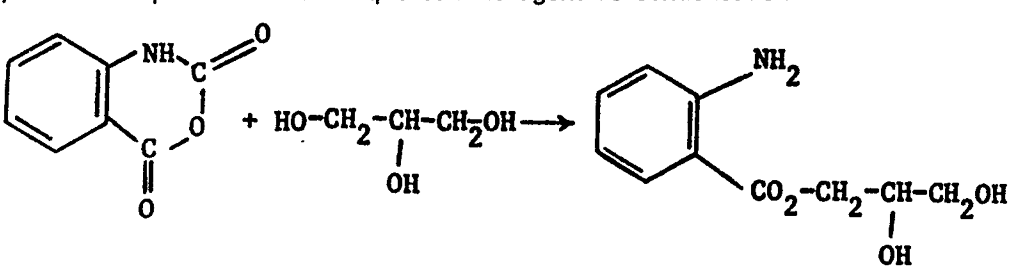

Ce procédé est décrit dans le brevet français déposé le 26 juillet 1977 au nom de la Demanderesse et ayant pour titre "Nouvel intermédiaire de synthèse: l'anthranilate de glycéryle et son procédé de préparation".

Ce procédé est conduit de préférence en utilisant un excès de glycérol comme solvant, on obtient donc l'anthranilate de glycéryle en solution dans le glycérol et cette solution peut être utilisée dans le procédé selon la présente invention sans avoir à isoler l'anthranilate, ce qui améliore encore le rendement global du procédé.

Ce procédé de préparation de la glafénine présente l'avantage de n'utiliser que des produits largement accessibles dans le commerce et peu coûteux.

Ainsi, l'anhydride isatoïque qui connaît un développement industriel en tant qu'intermédiaire de synthèse dans l'industrie des colorants peut, grâce à des nouveaux procédés de synthese, être obtenu à des prix très inférieurs aux prix de l'acide anthranilique qui constitue le produit de base dans les synthèses connues de la glafénine.

En outre, ce procédé de synthèse permet d'accroître notablement les rendements en glafénine par rapport aux procédés connus, notamment en limitant le nombre des étapes de réaction.

L'exemple suivant est destiné à illustrer le mode de mise en oeuvre préféré du procédé selon la présente invention.

## Exemple

*Préparation de l'$\alpha$ - monoglycéride de la 4(2' - carboxyphénylamino) - 7 - chloro - quinoléine* (glafénine).

Dans un réacteur de 100 l on introduit 29,3 kg de glycérol (321 moles) et on ajoute 157 g de soude en pastilles (3,9 moles) puis 5,1 kg d'anhydride isatoïque (31 moles).

On chauffe lentement, le gaz carbonique formé est aspiré par l'intermédiaire d'une pompe dont le débit est de 10 $m^3$/heure; la température est maintenue environ 1 heure entre 60 et 90°C.

A ce stade on obtient une solution d'anthranilate d'$\alpha$-glycéryle dans le glycérol.

A cette solution on ajoute 66,35 l d'acide chlorhydrique 0,5 N et 5,2 kg de 4,7 - dichloro - quinoléine recristallisée, la température est ramenée aux environs de 80°C pendant 40 minutes environ.

Le milieu réactionnel est ensuite refroidi à 20°C et transvasé dans une cuve de 200 l dans laquelle il est traité lentement par 60 l d'une solution aqueuse de soude N puis la neutralisation est terminée par addition de bicarbonate de soude.

La glafénine précipite lentement, le mélange réactionnel est envoyé par l'intermédiaire d'une pompe DELASCO dans une essoreuse. Le solide ainsi récupéré est remis en suspension dans 100 l d'eau.

La purification est effectuée par retour à une solution de chlorhydrate de glafénine et reprécipitation de la gléfénine base en opérant de la façon suivante: à la suspension aqueuse on rajoute 2830 $cm^3$ d'HCl de densité 1,18, le chlorhydrate formé se dissout, on filtre sur filtre monodisque, on rajoute de la soude N, puis on termine la neutralisation par une solution de bicarbonate de soude.

Le précipité est séparé comme précédemment par essorage; la glafénine brute ainsi obtenue a, après séchage, un point de fusion de 163°C.

Une recristallisation dans 100 l de chloroforme et 4 l d'éthanol à 95° permet une purification complémentaire.

Après séchage à l'étuve sous vide la glafénine est obtenue avec un rendement variant selon les cas de 65 à 75% par rapport à la 4,7 - dichloro - quinoléine avec un point de fusion instantané au bloc Kofler de 169—170°C.

Caractéristiques de la glafénine ainsi obtenue:

Formule brute: $C_{19}H_{17}Cl\ N_2O_4$.

Masse moléculaire: 372,8.

L'analyse élémentaire est en accord avec les normes traditionnellement exigées.

La teneur résiduelle en chloroforme est inférieure à 1.000 ppm.

Chromatographie en couche mince:

—support: silice Merck F 254

—révélateur: lampe à ultra-violets ou vapeurs d'iode

—solvant:

a) acétate d'éthyle, Rf $\simeq$ 0,4

b) butanol-acide acétique-eau, 6/2/2/. Rf = 0,66.

Spectrographie infra-rouge:

Appareil Perkin Elmer modèle 177.

Pastilles KBr.

Bandes d'absorption caractéristiques:

$\nu_{C=O}$ (ester) à 1680 $cm^{-1}$

$\nu_{C=C}$ (aromatiques) 1580 et 1620 $cm^{-1}$

$\nu_{OH}$ et $\nu_{NH}$ bande large entre 3100 et 3500 $cm^{-1}$.

Ce spectre est superposable à celui d'un authentique chimique obtenu différemment.

La présente invention concerne également, à titre de médicament, la glafénine obtenue par la mise en oeuvre du procédé selon la présente invention.

## Revendications

1. Procédé de préparation de l'$\alpha$-monoglycéride de 4(2' - carboxyphénylamino) - 7 - chloro - quinoléine, caractérisé en ce que:

1. on condense le glycérol sur l'anhydride isatoïque en milieu basique pour obtenir l'anthranilate de glycéryle, et

2. on condense l'anthranilate de glycéryle avec la 4,7 - dichloro - quinoléine et on récupère le produit désiré.

2. Procédé selon la revendication 1, caractérisé en ce que le glycérol est condensé sur l'anhydride isatoïque dans un excès de glycérol et que la solution réactionnelle obtenue est utilisée directement pour la condensation avec la 4,7 - dichloro - quinoléine.

3. Procédé selon l'une des revendications 1 et 2, caractérisé en ce que l'on opère la deuxiéme condensation en milieu chlorhydrique.

4. Procédé selon l'une des revendications 1 à 3, caractérisé en ce que l'on récupère le produit désiré en précipitant l'$\alpha$ - monoglycéride de 4(2' - carboxyphénylamino) - 7 - chloro - quinoléine par neutralisation du milieu réactionnel.

## Patentansprüche

1. Verfahren zur Herstellung des $\alpha$-Monoglycerids des 4(2' - Carboxyphenylamino) - 7 - chlorchinolin, dadurch gekennzeichnet, daß man

1. Glycerin mit Isatoïcsäureanhydrid in basischem Medium zu dem Anthranidsäureglycerinester kondensiert, und

2. den Anthanilsäureglycerinester mit 4,7-Dichlorchinolin kondensiert, und das gewünschte Produkt abtrennt.

2. Verfahren nach Anspruch 1, dadurch gekennzeichnet, daß man das Glycerin mit dem Isatoïcsäureanhydrid in Überschuss Glycerin kondensiert und die erhaltene Reaktionslösung direkt zur Kondensation mit 4,7-Dichlorchinolin verwendet.

3. Verfahren nach Anspruch 1 und 2, dadurch gekennzeichnet, daß man die zweite Kondensation in salzsaurem Medium durchführt.

4. Verfahren nach einem der Ansprüche 1 bis 3, dadurch gekennzeichnet, daß man das gewünschte Produkt abtrennt, indem man das $\alpha$-Monoglycerid des 4(2' - Carboxyphenylamino) - 7 - chlorchinolins durch Neutralisieren des Reaktionsmediums ausfällt.

## Claims

1. A process for the preparation of 4(2' - carboxyphenylamino) - 7 - chloro - quinoline - $\alpha$ - monoglyceride, characterised in that:

(i) glycerol is condensed on isatoic anhydride in a basic medium in order to obtain glyceryl anthranilate, and

(ii) glyceryl anthranilate is condensed with 4,7-dichloroquinoline and the desired product is recovered.

2. A process according to claim 1, characterised in that glycerol is condensed on isatoic anhydride in an excess of glycerol and that the resulting reaction solution is used directly for condensation with 4,7 - dichloro - quinoline.

3. A process according to one of claims 1 and 2, characterised in that the second condensation step is carried out in a hydrochloric medium.

4. A process according to one of claims 1 to 3, characterised in that the desired product is recovered by precipitating 4(2' - carboxyphenylamino) - 7 - chloroquinoline - $\alpha$ - monoglyceride by neutralising the reaction medium.